# EUROPEAN PATENT APPLICATION

(11) **EP 4 782 022 A1**
(43) Date of publication of application: **29.07.2026**
(21) Application number: 25154400.3
(22) Date of filing: 28.01.2025
(51) Int. Cl.: A61L 29/08, A61L 29/16

(54) **PROCESS AND APPARATUS FOR FLOW COATING A MEDICAL DEVICE**

(71) Applicant: CM4Cure s.a., 4000 Liège (BE)
(72) Inventor: OURY, Cécile, Liège (BE); IERMANO, Fabio, Liège (BE); AQIL, Abdelhafid, Liège (BE)
(74) Representative: Barker Brettell LLP

(57) **Abstract**

A process and apparatus for automatic flow coating internal and external surfaces of a medical device, particularly catheter, more particularly intravascular catheter.

## Description

### FIELD OF THE INVENTION

The present invention relates to a process and apparatus for flow coating a medical device, particularly a catheter, more particularly an intravascular catheter.

The present invention also relates to a process and apparatus for flow coating of a medical device with bioactive molecules, therapeutic molecules or drugs.

### BACKGROUND OF INVENTION

Process and apparatus for coating a medical device are known in the art. They are generally based on dip coating or immersion of the medical device in a coating solution. But, if applying a coating on the external surface of a medical device is relatively easy; applying a coating in an internal cavity, such as for example inside one or more lumen of a catheter may generate multiple problems such as bubbles formation, difficulty to fill the coating solution, making the coating, providing a uniform coating, particularly with intravascular catheter of small diameter size.

Particularly for catheter of small diameter, it is not possible to apply a viscous coating solution such as with heparin or anticoagulant agent to prevent blood clot inside a lumen of catheter.

CN104307690, CN118577439, CN106423719 disclose systems for vertically dip coating multiple catheters by immersing them using an automated lifting mechanism, followed by curing with a light source. But, while it may be possible to coat both internal or inner and external or outer surfaces via dipping, provided that the catheter diameter is large enough to allow capillarity; there is uncertainty about how the light source effectively reaches the inner surfaces of the catheters.

CN113058804 discloses a multiple drug-containing catheters coating by immersing them in a coating container connected to a drug storage tank. A complex gas pumping device made of gas storage cylinder, pipes and pumps communicates with the drug storage tank and can pump gas into the drug storage tank or pump gas out of the drug storage tank. Squeezing force of the gas on the liquid may occurs.

CN117324205 describes a system that can selectively coat the external or outer or internal or inner surfaces of catheters using a pump directly attached to the coating medium reservoir, along with a reflux chamber and pipe. The system is used for lubricating coating only.

US12064566B1 discloses a coating apparatus inserting a tube in a coating solution before or simultaneous with a step of drawing the coating solution into the lumen of the tube. This allows the outside surface of the tube to be coated in the same step as the lumen but the apparatus is only used for lubricious coating.

Process and apparatus for flow coating are also known in the art. Flow coating is generally known as an automated method of applying liquid coatings on a substrate using gravity to generate coating flows under controlled gravity over the substrate, distributing it evenly over the surface. But achieving consistent coating thickness can be challenging, especially with very thin layers.

Flow coating is also disclosed in KR20240161744 wherein the process is applied on a substrate using gravity to flow coating resin from top to bottom. The flow coating resin that flows down to the bottom is pumped back up to the top to be reused.

US2021/0335585 discloses a continuous flow system and method for coating a substrate, particularly a vacuum lock for coating substrates. The system is not adapted for coating internal and external surfaces of a medical device.

We have now found an easy-to-use process and apparatus that can advantageously flow coat simultaneously internal or inner and external or outer surfaces of a medical device, particularly a catheter; more particularly an intravascular catheter of small diameter size. The process and apparatus can also advantageously be carried out in horizontal or vertical position and does not rely on gravity.

### SUMMARY OF INVENTION

The object of the present invention is to provide an automated process and apparatus for flow coating medical device, particularly for intravenous catheter, enabling simultaneous flow coating of both external or outer and internal or inner surfaces of the medical device at a continuous and constant flow of a coating solution. It does not rely on a lifting mechanism, dip coating, or a light source for curing. No gas diffusion is required neither to coat drugs onto the surface of the medical device. Furthermore, a single pump and reservoir can monitor multiple catheters, reducing system complexity, space requirements, and cost.

The object of the invention is also to provide an automatic process and apparatus for flow coating medical device, particularly for intravenous catheter; enabling coating of both external or outer and internal or inner surfaces of the medical device with bioactive molecules, therapeutic molecules or drugs, and further continuous flow coating of a coating solution on both external or outer and internal or inner surfaces of the medical device.

### BRIEF DESCRIPTION OF THE FIGURES

The figures which are incorporated and constitute a part of the specification, illustrate embodiment of the invention and serve to explain the principles of the disclosure.
Fig.1 shows 1a) a side view of an assembly of a coating tube with a bottom (BE) endcap and a tip(TE) endcap; 1b) a section view of the same assembly according to a preferred embodiment with a catheter inside the cavity of the coating tube; 1c shows a front view of the assembly according to a preferred embodiment with a 2-lumen catheter inside the cavity of the coating tube
Fig.2 shows a top endcap wherein G is an outlet hole for outflow of the coating solution and F is a slit to connect the endcap to the coating tube
Fig.3 shows a bottom endcap wherein C is an inlet hole for inlet of the coating solution; D is a hole wherein the medical device is inserted; E is a slit to connect to the coating tube.
Fig.4 shows a butterfly clamp designed according to a preferred embodiment for clamping a catheter hub in the coating tube.
Fig.5 shows a clamp at the top of the coating tube, designed according to a preferred embodiment, which is able to keep a catheter straight and stretched to prevent any contact with the coating tube, enabling a uniform coating.
Fig.6 illustrates a schema of C1 coating cycle. C1, C2, C3, C4 represent 4 coating solutions or dispersion, V1 and V2 are two automated system of valves such as Rondelo 6-way valves to switch between C1, C2, C3, C4 solutions; W represents a flushing liquid such as for example water and Man represents a manifold
Fig.7 illustrates a C1 draining cycle with C1 coating solution
Fig.8 shows a schematic illustration of a preferred embodiment with dopamine solution as primer coating (C1), a polymer bearing primary or secondary amine groups, preferably poly-(allylamine hydrochloride) (PAH) as second coating solution (C2) and nanogel suspension as third coating solution (C3).

### DETAILLED DESCRIPTION:

**According to a first aspect** of the invention, there is provided a process for automatic flow coating of a medical device comprising the following steps:
a) Optionally applying a drug formulation as pre-treatment of both internal or inner and external or outer surfaces of the medical device;
b) Inserting the optionally pre-treated medical device into a coating tube;
c) Securing the medical device inside the coating tube by clamping the coating tube with a bottom endcap BE and a top endcap TE;
d) Simultaneously flow coating both internal or inner and external of outer surfaces of the medical device into the coating tube by continuous **injection** into the coating tube of a first coating solution at a continuous and constant flow from the bottom endcap to top endcap;
e) Draining the coating solution from the medical device at a continuous and constant flow from the top endcap to the bottom endcap;
f) Optionally repeating step d) and e) with subsequent coating solutions;
g) drying the resulting flow coated medical device.

The medical device includes, but is not limited to, any device, tool, instrument, implant, or the like, relating to medicine or the practice of human or veterinary medicine, or intended for use to heal or treat a disease or condition. A medical device may include all natural and synthetic materials and both fibrous and non-fibrous materials. For example, the materials may be comprised of a metal, plastic, glass, ceramic, rubber, polymer, composite material or any other material or combination of materials. Exemplary medical devices include, but are not limited to, any kind of catheter such as intravenous catheter; cannulae; needles; stents of any size, shape, or placement; coils of any size, shape, or placement; endotracheal tubes; gastroenteric feeding tubes; arteriovenous shunts and the like.

In a preferred embodiment the medical device is a catheter, preferably an intravascular catheter

Catheters are flexible tubes used to deliver fluids into or withdraw fluids from the body. They may be made of silicone (polydimethylsilixane), latex, high density polyethylene, elastomers, polyvinylchloride (PVC), polytetrafluoroethylene (PTFE), and the like.

Intravascular catheter is a catheter of small dimension such as Midlines, PICC, and Hickman catheters having an outer diameter between 0.67 and 11.33mm corresponding to a range of 3 to 34 FR.

In a more preferred embodiment, the catheter is a multi-lumen catheter, most preferably a 2-lumen catheter.

***In optional step a), a pre-treatment of the medical device is provided by applying a drug formulation on both internal or inner and external or outer surfaces of the medical device*** at temperature controlled between 20 and 45°C; preferably between 30 and 40°C, most preferably 37°C.

The optional pre-treatment of the medical device may be applied by several technics such as immersion of the device into the drug formulation; spraying of the drug formulation or flow coating of the drug formulation on both internal or inner and external or outer surfaces of the medical device.

The drug formulation is obtained by solubilization or dispersion of the drug in water or in an organic solvent such as for example alcohol, ester, ethers, ethyl acetate and the like; or a mixture thereof. In a preferred embodiment, the drug is solubilized or dispersed in alcohol, preferably ethanol.

The drug may be an antibacterial, antifungal, anti-platelet, anti-coagulant, antithrombotic, anti-calcification agent or a combination thereof.

Antibacterial agents include but are not limited to amikacin, gentamicin, kanamycin, neomycin, netilmicin, tobramycin, paromomycin, streptomycin, spectinomycin, geldanamycin, herbimycin, rifaximin, loracarbef, ertapenem, dorpenem, imipenem/cilastatin, meropenem, cefadroxil, cefazolin, cefalotin, cephalexin, cefaclor, cefamandole, cefoxitin, cefproxil, cefuroxime, cefixime, cefdinir, cedfitoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, daibavancin, oritavancin, clindamycin, lincomycin, daptomycin, azithromycin, clarithromycin, dirithromycin, erythromycin, roxithromycin, troleandomycin, telithromycin, spiramycin, aztreonam, furazolidone, nitrofurantoin, linezolid, amoxicillin, ampicillin, piperacillin, ticarcillin, bacitracin, colistin, polymyxin B, ciprofloxacin, enoxacin, gatifloxacin, gemifloxacin, levoflaxicin, lomefloxacilin, moxifloxacin, nalidixic acid, norfloxacin, ofloxacin, mafenide, sulfacetamide, sulfadizine, silver sulfadizine, sulfadimethoxine, sulfamethizole, sulfamethoxazole, sulfanilimide, sulfisoxazole, trimethoprim-sulfamethoxazole, sulfonamidochrysoidine, demeclocyline, doxycycline, minocycline, oxytetracycline, tetracycline, clofazimine, dapsone, rifampicin, rifabutin, arspehnamine, chloramphenicol, fosfomycin, metronidazole, thiamphenicol, tigecycline, tinidazole, and trimethoprim.

Antifungal agents include but are not limited to clotrimazole, econaole, miconazole, terbinafine, fluconazole, isavuconazile, itraconazole, ketoconazole, nystatin, Posaconazole, voriconazole), but also polyenes such as amphotericin B and chlorhexidine (CHX) belonging to the Bisbiguanide antifungal agents having both antifungal and antibacterial activity;

Anti-platelet agents include, but are not limited to, irreversible cyclooxygenase inhibitors such as aspirin and triflusal (Disgren), adenosine diphosphate (ADP) receptor inhibitors such as clopidogrel (Plavix), prasugrel (Effient), ticagrelor (Brilinta), ticlopidine (Ticlid), Phosphodiesterase inhibitors such as cilostazol (Pletal), Protease-activated receptor-1 (PAR-1) antagonists such as vorapaxar (Zontivity), glycoprotein IIB/IIIA inhibitors (intravenous use only) such as abciximab (ReoPro), eptifibatide (Integrilin), tirofiban (Aggrastat), Adenosine reuptake inhibitors such as dipyridamole (Persantine), thromboxane inhibitors, thromboxane synthase inhibitors and thromboxane receptor antagonists such as terutroban, glycoprotein VI inhibitors such as Revacept, glycoprotein Ib inhibitors, and von Willebrand factor inhibitors.

Anti-coagulants include, but are not limited, to acenocoumarol, argatroban, coumatetralyl, dicoumarol, ethyl biscoumacetate, phenprocoumon, warfarin, clorindione, dipjenadione, phenindione, ticlomarol, bemiparin, certoparin, ardeparin, dalteparin, enoxaparin, nadroparin, parnaparin, reviparin, dabigatran, apixaban, betrixabaan, darexaban, edoxaban, otamixaban, rivaroxaban, alteplase, danaparoid, tinzaparin, and fondaparinux.

Antithrombolytic agents include, but are not limited to, Dabigatran, Desirudin, Apixaban, Betrixaban, Edoxaban, Fondaparinux, Heparin, Dalteparin, Enoxaparin, Tinzaparin, Warfarin
Anti-calcification agents include, but are not limited to, bisphosphonates, aluminium salts, glutaraldehyde, amino oleic acid, and metalloproteinase inhibitors.

In a preferred embodiment the drug formulation comprises an antibacterial and anti-platelet agents represented by

### Triazolo(4,5-d)pyrimidine derivative of formula (I)

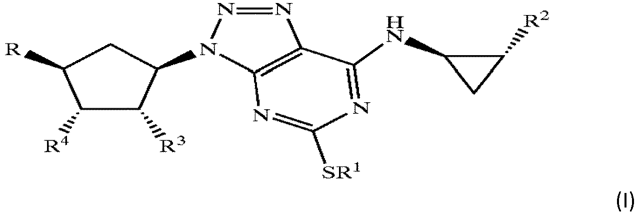

wherein R₁ is C ₃₋₅ alkyl optionally substituted by one or more halogen atoms; R₂ is a phenyl group, optionally substituted by one or more halogen atoms; R₃ and R₄ are both hydroxyl; R is XOH, wherein X is CH₂, OCH₂CH₂, or a bond;
or a pharmaceutical acceptable salt or solvate thereof, or a solvate of such a salt provided that when X is CH₂ or a bond, R₁ is not propyl; when X is CH₂ and R₁ CH₂CH₂CF₃, butyl or pentyl, the phenyl group at R₂ must be substituted by fluorine; when X is OCH₂CH₂ and R₁ is propyl, the phenyl group at R₂ must be substituted by fluorine;

In a most preferred embodiment, the antibacterial and anti-platelet formulation comprises (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called triafluocyl and represented by formula II 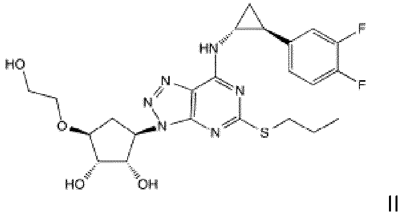 or 1S,2R,3S,4R)-4-[7-[[(1R,2S)-2-(3,4-Difluorophenyl)cyclopropyl]amino]-5-(propylthio)-3H-1,2,3-triazolo[4,5-d]pyrimidin-3-yl]-1,2,3-cyclopentanetriol also called Fluometacyl represented by formula (III)

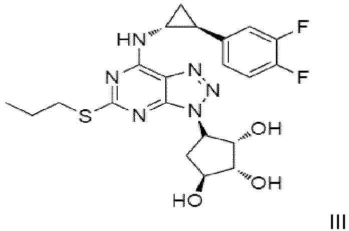

The preferred embodiment with Triazolo(4,5-d)pyrimidine derivative , preferably triafluocyl or fluometacyl that are anti-platelet agents may advantageously provide a medical device, particularly an intravascular catheter with anti-platelet effect that avoid blood clot inside lumen of catheter.

The time of pre-treatment when using immersion of the medical device in drug formulation is dependant of temperature and may vary according to the selected drug. In a preferred embodiment, it is preferably between 0,5 to 1 hour for triafluocyl or fluometacyl in ethanol solution at 37°C.

In another preferred embodiment, the drug formulation comprises an antimicrobial formulation preferably a combination of Triazolo(4,5-d)pyrimidine derivative of formula (I)
together with one antibacterial agent selected from the group consisting of fluoroquinolone and pharmaceutical acceptable salts thereof, tetracycline and pharmaceutical acceptable salts thereof or azole and pharmaceutical acceptable salts thereof.

### In step b, the medical device is inserted into a coating tube.

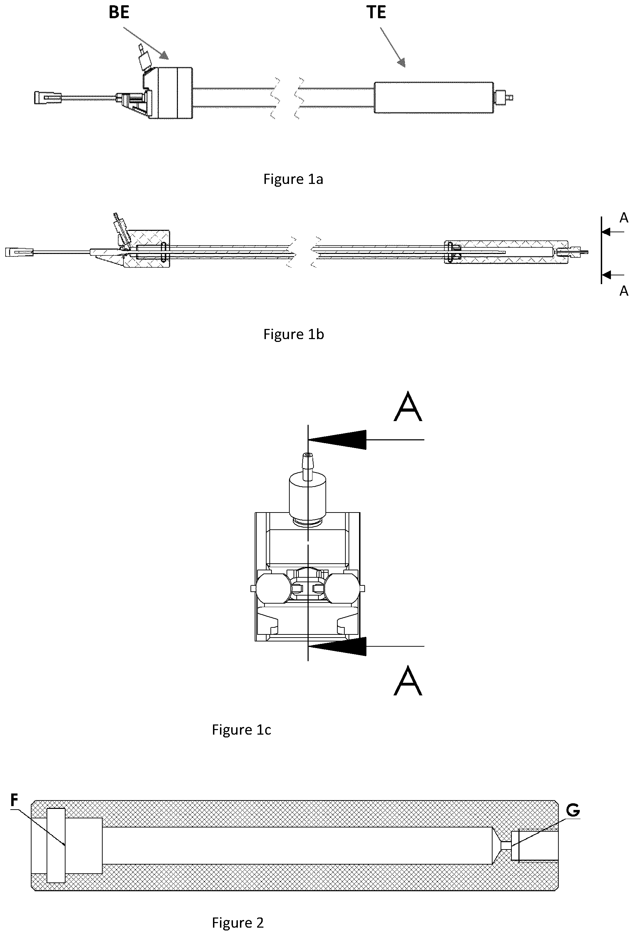

The coating tube is illustrated in figure 1 according to a preferred embodiment wherein the medical device is a catheter, preferably a 2-lumen catheter. Figure 1 shows a side view of the coating tube (Figla), a section view of the tube with the catheter inside the cavity of the coating tube (Fig 1b) and a front view A-A of the coating tube with the 2-lumen catheter inside the tube.

The coating tube comprises an assembly of a tube and two endcaps.

The tube is made of glass, polycarbonate or any other preferably transparent material. The coating tube is designed to facilitate insertion of a medical device.

The design of the coating tube is made longer than the medical device, allowing both simultaneous internal and external flow of the coating solution to merge after coating of both internal or inner and external or outer surfaces, beyond the medical device tip. This assembly simplifies the process, reduces the number of outlets and minimizes dead volume

The assembly tube-endcaps as illustrated in figure 1 is easy to construct, leak-free, and does not require permanent connections. The assembly provides flexibility to coat different types of medical devices such as catheters by adjusting dimension of the coating tube, allowing it to accommodate various catheter sizes. A low-dead-volume design of the endcaps and tube reduces the amount of coating solution required per medical device, which in turn lowers costs.

***In step C, the medical device is secured inside the coating tube by clamping the coating tube with a bottom endcap and a top endcap.***

The medical device is clamped within the coating tube at the bottom endcap and at the top of the coating tube which keep the medical device straight and stretched to prevent any contact with the coating tube walls and enabling a uniform coating.

The endcaps are made for example from polytetrafluoroethylene (PTFE), polyetherketone (Peek), Acrylonitrile butadiene styrene(ABS), silicone, low density polyethylene (LPE), thermoplastic elastomer TPE), polyvinylchloride (PVC) and the like.

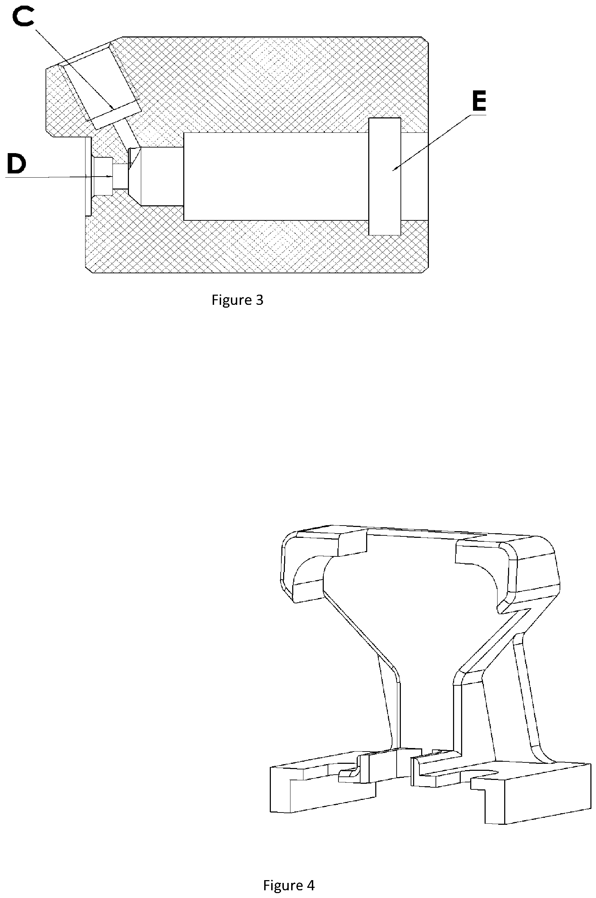

The bottom endcap is illustrated in figure 3 and comprises an inlet hole C for entry of the coating solution, a hole D for insertion of the medical device and a slit E for connection with the coating tube

The top endcap is illustrated in figure 2 and comprises a slit F for connection with the coating tube and an outlet hole G for outlet of the coating solution.

An example of bottom endcap according to a preferred embodiment is the butterfly clamp as illustrated in figure 4. Such butterfly clamp is specially designed to clamp a hub of a 2-lumen catheter.

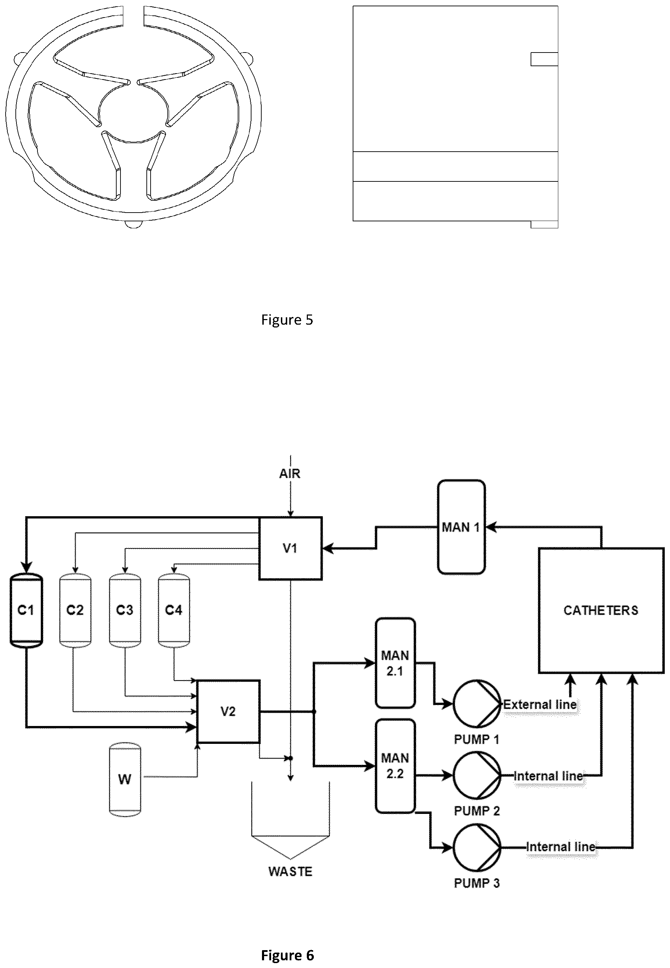

An example of top clamp is illustrated at figure 5.

For longer medical device such as catheters, two clamps may not be sufficient to maintain alignment and avoid contact with the tube. In such a case, a guide clamp is added to keep the medical device centred in the coating tube.

In a preferred embodiment, the coating tube comprising the medical device is further connected to coating solutions reservoirs via a circulation arrangement made of pipes, valves (V), manifolds (man) and multiline pumps (V).

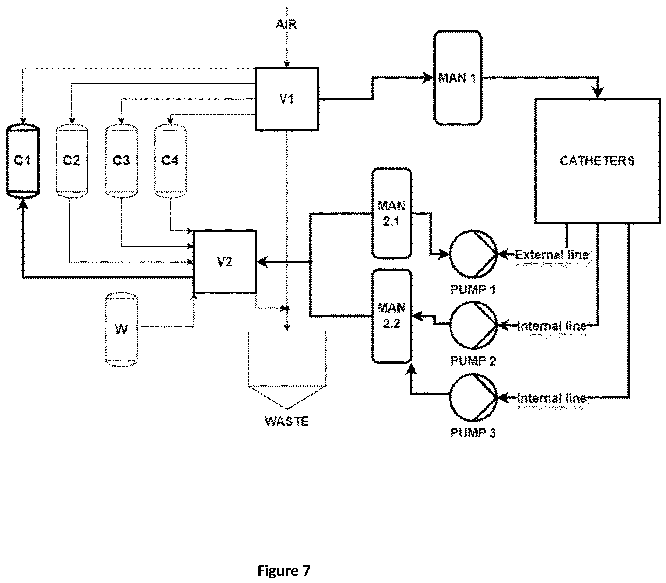

In a most preferred embodiment, the circulation arrangement is represented in figure 6 and 7 respectively for injection of coating solution into the coating tube and for draining the coating solution from the coating tube. An external line represents pipes or tubes respectively supplying or draining the coating solution to or from the coating tube embedding the medical device; whereas the internal line represents pipes or tubes supplying or draining internal cavity of the medical device as for example the lumen in a multi-lumen catheter according to an exemplary embodiment.

Particularly for a catheter according to an exemplary embodiment, an internal line connection to the coating solution reservoir is made through the catheter s' hub; whereas an external line connection is done through the bottom endcap of the coating tube as illustrated in figure 3.

The circulation arrangement may also comprise a manifold-in with incoming tubes and manifold-out with outgoing tubes, respectively providing through pump channels, different coating solutions into the coating tube from a coating solution reservoir or returning the coating solution from the coating tube to the corresponding coating solution reservoir.

***In step d: Simultaneously flow coating both internal or inner and external or outer surfaces of the medical device into the coating tube by continuous injection into the coating tube of a first coating solution of a continuous and constant flow from the bottom endcap to top endcap.***

It means that the simultaneous flow coating of the internal or inner and external or outer surfaces of medical devices is achieved through an inflow of the coating solution into the coating tube and internal cavity of the medical device.

The continuous and constant flow for injection into the coating tube with a first coating solution is divided into internal flow for coating internal or inner surface of the medical device and external flow for coating the external or outer surface of the medical device. The internal flow goes from the bottom endcap to the top endcap at a constant flow, preferably at a laminar flow. The external flow for coating the external or outer surface of the medical device, goes from the bottom endcap to top endcap at constant flow, preferably at a laminar flow. This advantageously prevents dripping and eliminates trapped air bubbles.

The process for automatic flow coating the medical device may also be carried out at controlled temperature between 20 and 45°C; preferably at room temperature.

In a preferred embodiment, the continuous and constant flow rate for injection the coating solution into the coating tube is between 0.01 and 1ml/min, preferably between 0.05 and 1ml, more preferably 0.05 ml/min for the internal flow and more preferably 0.5ml/min for the external flow.

The flow coating time, at room temperature may vary between 10 minutes to 5 hours depending of the coating solution or dispersion.

In another preferred embodiment, and in order to improve the wetting of the internal or inner and external or outer surfaces of the medical device to the coating solutions; the coating tube may be preliminary filled with the coating solution at a flow rate between 0.5 and 5ml/min, preferably 0.65 ml/min for the internal flow and 5 ml/min for the external flow.

In a most preferred embodiment, the first coating solution is a primer, preferably dopamine.

***In step e) Draining the coating solution from the medical device of continuous and constant flow from the top endcap to the bottom endcap.***

In a preferred embodiment, the continuous and constant flow rate for draining the coating tube from the coating solution is between 0.5 and 5ml/min, preferably 0.65 ml/min for the internal flow and 5 ml/min for the external flow.

***In step f) Optionally repeating step d) and e) with subsequent coating solutions.***

In a preferred embodiment, repeating the step of simultaneous coating of both internal and external surfaces of the medical device into the coating tube, by injecting into the coating tube a second coating solution at a continuous and constant flow from the bottom endcap to the top endcap and repeating step d) of draining the second coating solution from the coating tube.

Both steps of injection and draining may be further repeated for a third or further coating solution or dispersion.

The second coating solution is preferably a polymer bearing primary or secondary amine groups, preferably poly-(allylamine hydrochloride) (PAH) solution.

The third flow coating solution is a nanogel suspension, preferably made of polyquinone, more preferably made of a poly(vinylquinone), most preferably made of a poly(methacrylamide)-bearing quinone groups of formula (IV) 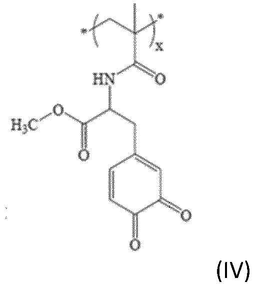 wherein x is an integer > 1, preferably x is between 1 and 100; said poly(methacrylamide) been crosslinked with a polymer bearing primary or secondary amine groups.

In the most preferred embodiment the polymer bearing primary or secondary amine groups in the nanogel solution is poly-(allylamine hydrochloride) (PAH) solution.

The crosslinking of poly(methacrylamide) or poly(vinylquinone with a polymer bearing primary or secondary amine groups, preferably poly-(allylamine hydrochloride) (PAH) is described in WO2024188572.

Alternatively, the third flow coating solution is the polyquinone, preferably the poly(methacrylamide)-bearing quinone groups.

***In step g) drying the resulting flow coated medical device*** with a dryer system to remove water from the medical device.

The process for flow coating medical device can automatically and simultaneously injecting coating solution by inflow into the internal or inner surface and external or outer surface of a large number of medical devices, preferably from 1 to 24 medical devices, particularly catheters, more particularly intravascular catheter, most particularly 2 lumen catheters

**In another aspect the of the invention,** there is also provided an apparatus for automatic flow coating of a medical device comprising a coating tube made of an assembly tube-endcaps with the tube connected to hole E of a bottom endcap, said bottom endcap further comprising a hole D for inserting the medical device and an inlet hole C for inlet of the coating solution; the tube is further connected to hole F of a top endcap said endcap having also an outlet hole G for outlet of the coating solution.

The coating tube may be secured in vertical, horizontal or any other positions. In a preferred embodiment the coating tube is in vertical position.

In a preferred embodiment the medical device is a catheter, preferably an intravascular catheter, more preferably a multi -lumen catheter, most preferably a 2-lumen catheter.

In a preferred embodiment, the medical device is secured inside the tube by a clamp at the bottom endcap and by top clamp at the top endcap. The medical device may also be secured by a third clamp or guide clamp to keep the medical device centred in the coating tube.

In a most preferred embodiment, the bottom endcap is a butterfly endcap specially designed to clamp a catheter's hub.

In another preferred embodiment, the bottom endcap is connected to a manifold IN with incoming tubes or to a manifold OUT with outgoing tubes, respectively providing coating solution into the coating tube or returning the coating solution from the coating tube to the corresponding coating solution reservoir.

The apparatus is also equipped of a temperature-controlled system. The temperature-controlled system may be for example a controlled water bath, a heat exchanger, a heating device embedding the coating tube and the like.

The apparatus for flow coating medical device is also adapted to automatically and simultanrously injecting by inflow into the internal or inner surface and external or outer surface of a large number of medical devices.

In a preferred embodiment the apparatus is carrying out from 1 to 24 medical device, particularly catheters, more particularly intravascular catheter, most particularly 2 lumen catheters.

The invention will be further described by means of examples, with references to the figures.

**Example 1:** Process for flow coating a catheter with bioactive molecules, therapeutic molecules or drugs also called APis.

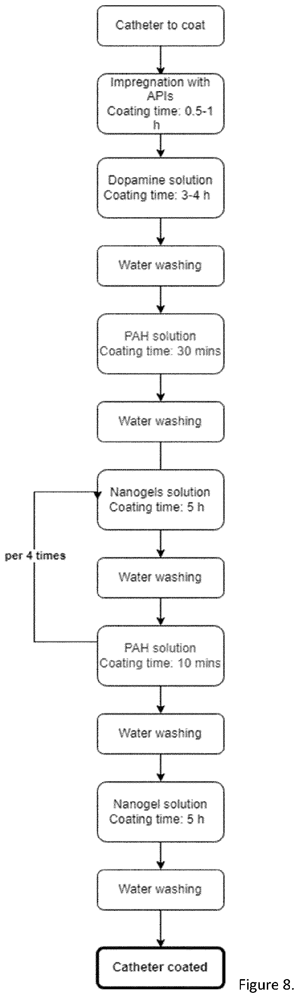

Figure 8 illustrates a flow chart of the flow coating process, wherein each step is performed in a bottom-to-top flow in the coating tube. The catheter is positioned vertically in the coating tube with the hub at the bottom and the tip at the top.

***As first step,*** the catheters are pre-treated by immersion in a drug formulation during 1 hour in a temperature-controlled bath at 37°C. The catheters are submerged in an ethanol - fluometacyl. After the pre-treatment step, the catheter is dried at room temperature for one night at least, to assure that the ethanol is completely evaporated.

***As a second step,*** simultaneously coating both inner or internal and outer or external surfaces of the pre-treated catheter by continuously injecting or flowing a dopamine solution through the coating tube and the lumens of the catheters for 3 to 4 hours. The dopamine solution is maintained at constant flow rates, moving from the bottom endcap to the top endcap. The internal flow rate is 0.05 ml/min; whereas the external flow is 0.5 ml/min.

***As a third step,*** water washing with a water flush during 10 minutes and an air flush during 5 minutes at an internal flow rate of 0.65ml/min and at an external flow rate at 5 ml/min.

***As fourth step,*** simultaneously coating both the inner or internal and outer or external surfaces of the catheter obtained at step 3, by continuously flowing or injecting a PAH solution through the coating tube and the lumens of the catheters for 30 minutes. The solution is maintained at constant flow rates, moving from the bottom endcap to the top endcap. The internal flow rate is 0.05 ml/min; whereas the external flow is 0.5 ml/min

***As fifth step,*** water washing during 5 minutes at internal flow rate of 0.65ml/min and at an external flow rate of 5 ml/min.

***As sixth step,*** repeating 4 times the following sequences:
1- simultaneously flow coating both inner or internal and outer or external surfaces of the catheter into the coating tube by continuously flowing or injecting a nanogel suspension through the coating tube and the lumens of the catheter for 5 hours. The nanogel suspension is maintained at constant flow rates, moving from the bottom endcap to top endcap. The internal flow rate is 0.05 ml/min; whereas the external flow is 0.5 ml/min. The nanogel is a poly(methacrylamide) crosslinked with poly-(allylamine hydrochloride) (PAH) .
2- water washing during 5 minutes at an internal flow rate of 0.65 ml/min and at external flow rate of 5 ml/min.
3- simultaneously coating both inner or internal and outer or external surfaces of the catheter into the coating tube by continuously flowing or injecting PAH solution through the coating tube and the lumens of the catheter for 10 minutes. The PAH solution is maintained at a constant rates, moving from the bottom endcap to top endcap. The internal flow rate is 0.05 ml/min; whereas the external flow rate is 0.5 ml/min.

***As seventh step,*** water washing during 5 minutes at an internal flow rate of 0.65 ml/min and at external flow rate of 5 ml/min.

***As eight step,*** simultaneously coating both the inner or internal and outer or external surfaces of the catheter obtained at step seven, by continuously flowing or injecting a nanogel suspension through the coating tube and the lumens of the catheter for 5 hours. The nanogel suspension is maintained at constant flow rates, moving from the bottom endcap to the top endcap. The internal flow rate is 0.05 ml/min; whereas the external flow is 0.5 ml/min. The nanogel suspension is a poly(methacrylamide) crosslinked with poly-(allylamine hydrochloride) (PAH).

***As nineth step,*** water washing during 5 minutes at an internal flow rate of 0.65 ml/min and at external flow rate of 5 ml/min.

***As tenth step,*** drying the coated catheter to remove water from the catheter.

**Example 2:** reproducibility study of coating process considering the loading of API Fluometacyl on the catheters surface within three productions of twelve catheters each.

The process described in the present invention was applied to three production batches, each consisting of 12 one-lumen central venous catheters (CVCs) manufactured by Teleflex, using Fluometacyl as drug formulation to evaluate the reproducibility of the coating process. Table 1 presents the amount of Fluometacyl per centimeter loaded onto the catheter surfaces during the coating process for each catheter and production batch.

The content of Fluometacyl was determined using high-performance liquid chromatography (HPLC). An Arc HPLC system was utilized, consisting of a quaternary gradient solvent delivery system, a variable-volume injector, a temperature-controlled autosampler, a column thermostat, and a 2998 photodiode array detector.

**Table 1 Fluometacyl (Fluo) content (µg/cm] on catheters (cat.) surface within three productions (PRO) A, B, C of twelve catheters each.**

| **PRO** | | **Cat. 1** | **Cat. 2** | **Cat. 3** | **Cat. 4** | **Cat. 5** | **Cat. 6** | **Cat. 7** | **Cat. 8** | **Cat. 9** | **Cat. 10** | **Cat. 11** | **Cat. 12** |
|---|---|---|---|---|---|---|---|---|---|---|---|---|---|
| **A** | Fluo conte nt [µg/c m] | 13.3 | 12.1 | 11 | 9.8 | 10.4 | 9.6 | 13.1 | 13.4 | 12.9 | 11.5 | 13.5 | 10.2 |

| | | **Cat. 1** | **Cat. 2** | **Cat. 3** | **Cat. 4** | **Cat. 5** | **Cat. 6** | **Cat. 7** | **Cat. 8** | **Cat. 9** | **Cat. 10** | **Cat. 11** | **Cat. 12** |
|---|---|---|---|---|---|---|---|---|---|---|---|---|---|
| **B** | Fluo conte nt [gg/c m] | 12 | 13.5 | 13.2 | 14.3 | 11.3 | 11.5 | 14.3 | 16.2 | 11.4 | 15.1 | 14.5 | 11.6 |

| | | **Cat. 1** | **Cat. 2** | **Cat. 3** | **Cat. 4** | **Cat. 5** | **Cat. 6** | **Cat. 7** | **Cat. 8** | **Cat. 9** | **Cat. 10** | **Cat. 11** | **Cat. 12** |
|---|---|---|---|---|---|---|---|---|---|---|---|---|---|
| **C** | Fluo conte nt [µg/c m] | 10.7 | 9.8 | 10.1 | 9.6 | 9.9 | 11.4 | 11.1 | 11.6 | 10.9 | 10.7 | 10.3 | 11.1 |

From the analysis of this data, an intra-production standard deviation of 1.71 µg/cm was observed, with an average concentration of 11.86 µg/cm. Given a coefficient of variation of only 0.14 (a coefficient of variation < 1 indicates minimal variation within the sample population), it can be concluded that the process is consistent and capable of producing reproducible coatings both within and across production batches.

## Claims

1. A process for automatic flow coating of a medical device comprising the following steps:
a) Optionally applying a drug formulation as pre-treatment of both internal or inner and external or outer surfaces of the medical device;
b) Inserting the optionally pre-treated medical device into a coating tube;
c) Securing the medical device inside the coating tube by clamping the coating tube with a bottom endcap and a top endcap;
d) Simultaneously flow coating both internal or inner and external or outer surfaces of the medical device into the coating tube by continuous injection into the coating tube of a first coating solution at a continuous and constant flow from the bottom endcap to the top endcap;
e) Draining the coating solution from the medical device at continuous and constant flow from the top endcap to the bottom endcap;
f) Optionally repeating step d) and e) with subsequent coating solutions;
g) drying the resulting flow coated medical device.

2. The process according to claim 1 characterized-in-that the continuous and constant flow at step d) is between 0.01 and 1ml/min, preferably between 0.05 and 0.5ml/min, and the continuous and constant flow at step e) is between 0.5 and 5ml/min, preferably between 0.65 ml/min and 5ml/min.

3. The process according to claim 1 or 2 characterized-in-that the pre-treatment is conducted by immersion at a controlled temperature between 20° to 45°C, preferably 30 to 40°C, most preferably 37°C.

4. The process according to any one of claims 1 to 3 characterized-in-that
the drug formulation is an antibacterial and/or antifungal formulation and or antithrombotic formulation.

5. The process according to anyone of claims 1 to 4, characterized-in-that the first coating solution is a primer, preferably a dopamine solution.

6. The process according to anyone of claims 1 to 5 characterized-in-that the second and third coating solution at step f are respectively a polymer bearing primary or secondary amine groups, preferably poly-(allylamine hydrochloride) (PAH) solution.
and a polymer bearing quinone groups or a nanogel solution, preferably made of polyquinone, more preferably made of poly(methacrylamide )-bearing quinone groups or poly(vinylquinone), most preferably made of poly(methacrylamide )-bearing quinone groups.

7. The process according to claim 6 characterized-in-that the nanogel solution is a nanogel suspension made of a poly(methacrylamide)-bearing quinone groups of formula (1) 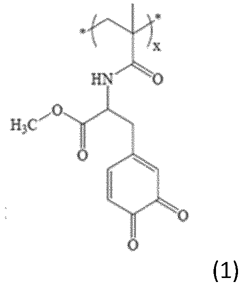 wherein x is an integer > 1, preferably x is between 1 and 100; said poly(methacrylamide) been crosslinked with a polymer bearing primary or secondary amine groups preferably poly-(allylamine hydrochloride) (PAH) solution.

8. The process according to anyone of claim 1 to 7 characterized -in-that the coating tube is vertically secured during the flow coating injection.

9. The process according to anyone of claim 1 to 8 characterized-in-that the medical device is a catheter, preferably an intravascular catheter, more preferably a multi-lumen catheter, most preferably a 2-lumen catheter.

10. The process according to claim 9 characterized-in-that the outer or external catheter size is between 0.67 and 11.33mm corresponding to a range of 3 to 34 FR

11. Apparatus for flow coating of a medical device characterized-in-that said apparatus comprises a coating tube connected to hole E of a bottom endcap, said bottom endcap further comprising a hole D for inserting the medical device and an inlet hole C for inlet of the coating solution; the coating tube is further connected to hole F of a top endcap said endcap having an outlet hole G for outlet of the coating solution.

12. The apparatus according to claim 11 characterized-in-that the medical device been secured inside the tube by a clamp at the bottom endcap and by a top clamp at the top endcap.

13. The apparatus according to claim 12 further comprising a guide clamp to keep the medical device centred in the coating tube.

14. The apparatus according to anyone of claims 11 to 13 characterized -in-that the bottom endcap is connected to a manifold-IN with incoming tubes or to a manifold-OUT with outgoing tubes, respectively providing coating solution into the coating tube from a reservoir or returning the coating solution from the coating tube to the reservoir.

15. The apparatus according to anyone of claims 15 to 18 characterized-in-that it further comprises a temperature-controlled system.
